## Europäisches Patentamt

## European Patent Office

## Office européen des brevets

(19)

(11) **EP 1 287 838 A2**

(12) **EUROPÄISCHE PATENTANMELDUNG**

(43) Veröffentlichungstag:
05.03.2003   Patentblatt 2003/10

(51) Int Cl.7: **A61M 1/16**

(21) Anmeldenummer: 02026631.8

(22) Anmeldetag: 26.05.1999

(84) Benannte Vertragsstaaten:
**DE ES FR GB IT**

(30) Priorität: **28.05.1998   DE 19823811**

(62) Dokumentnummer(n) der früheren Anmeldung(en)
nach Art. 76 EPÜ:
**99110193.2 / 0 960 625**

(71) Anmelder: **Fresenius Medical Care Deutschland
GmbH
61352 Bad Homburg v.d.H. (DE)**

(72) Erfinder:
• **Krämer, Mathias, Dr.
61381 Friedrichsdorf (DE)**

• **Johner, Christian, Dr.
61352 Bad Homburg (DE)**
• **Müller, Carsten, Dr.
97502 Euerbach (DE)**

(74) Vertreter: **Luderschmidt, Schüler & Partner
Patentanwälte,
John-F.-Kennedy-Strasse 4
65189 Wiesbaden (DE)**

Bemerkungen:
Diese Anmeldung ist am 29 - 11 - 2002 als
Teilanmeldung zu der unter INID-Kode 62
erwähnten Anmeldung eingereicht worden.

(54)    **Verfahren zur Erhöhung der Sicherheit für eine Blutbehandlungsvorrichtung**

(57)    Zur Erhöhung der Sicherheit einer extrakorporalen Blutbehandlungsvorrichtung, die über eine Regelung der Ultrafiltrationsrate in Abhängigkeit von dem Blutvolumen des Patienten verfügt, wird die Ultrafiltrationsrate UFR(t) während der Behandlung überwacht. Aus dem vorgegebenen Ultrafiltratgesamtvolumen $UFV_{ges}$ und der vorgegebenen Behandlungsdauer UFT wird ein oberer Grenzwert für die Ultrafiltrationsrate $UFR_{lim}$ bestimmt. Das Verfahren erlaubt die Limitierung der Ultrafiltrationsrate auf den oberen Grenzwert, so dass auch bei einer fehlerhaften Blutvolumenregelung eine Gefährdung des Patienten vermieden wird.

Fig.1

EP 1 287 838 A2

**Beschreibung**

Die Erfindung betrifft ein Verfahren zur Erhöhung der Sicherheit für eine extrakorporale Blutbehandlungsvorrichtung, insbesondere eine Hämodialyse-, Hämofiltrations- oder Hämodiafiltrationsvorrichtung

Zur Entfernung von harnpflichtigen Substanzen und zum Flüssigkeitsentzug werden beim chronischen Nierenversagen verschiedene Verfahren zur operativen Blutreinigung bzw. Blutbehandlung eingesetzt. Bei der Hämodialyse (HD) überwiegt der diffuse Stofftransport, während bei der Hämofiltration (HF) ein konvektiver Stofftransport über die Membran stattfindet. Eine Kombination aus beiden Verfahren ist die Hämodiafiltration (HDF).

Der Entzug von überschüssigem Körperwasser durch Ultrafiltration ist ein wesentlicher Bestandteil der Dialysetherapie. Dieser Vorgang führt meist zu einer Reduktion des Blutvolumens des Patienten. Bei einer zu starken Reduktion des Blutvolumens tritt symptomatische Hypotonie auf, eine häufige Begleiterscheinung der Dialysetherapie. Die Toleranz der Patienten gegen Volumenreduktion ist individuell sehr unterschiedlich; bei kritischen Patientenkollektiven, z.B. Diabetiker, Artherosklerosepatienten, treten die Blutdruckabfälle deutlich gehäuft auf.

Bei der heutigen Standardtherapie wird entweder eine feste Ultrafiltrationsrate UFR oder aber ein fester zeitlicher Verlauf der Rate (UF-Profil) vorgegeben. Eine Messung der Blutvolumenreduktion erfolgt nicht, und erst nach Auftreten von Problemen wird die Ultrafiltrationsrate manuell verringert und evtl. durch Infusionen Blutvolumen substituiert.

Es ist vorgeschlagen worden, das Blutvolumen des Patienten während der extrakorporalen Blutbehandlung zu überwachen und die Ultrafiltrationsrate derart einzustellen, dass Reduktionen des Blutvolumens, die hinsichtlich der Kreislaufstabilität des Patienten nicht tolerierbar sind, vermieden werden. Technische Voraussetzung für dieses Verfahren ist eine hinreichend genaue Sensorik zur Bestimmung des Blutvolumens.

Die Menge des während der Behandlung zu ultrafiltrierenden Volumens, d.h. des Ultrafiltratgesamtvolumens $UFV_{ges}$, sowie die Ultrafiltrationsrate UFR wirken sich, wie bereits erwähnt, auf die Flüssigkeitskompartiments des Körpers sowie auf die Kreislaufregulation aus. Der Vorgang der Ultrafiltration und somit auch die Blutvolumenregelung ist daher sicherheitskritisch. So kann bei unkontrolliertem Flüssigkeitsentzug von einigen l/h bereits nach einigen Minuten ein für die Kreislaufregulation kritisches Blutvolumen erreicht sein und ein schwerer Blutdruckabfall ausgelöst werden, der intensiv medizinische Maßnahmen erforderlich macht.

Bisher konnte nicht sichergestellt werden, dass auch im Falle einer Fehlfunktion der Sensorik und des Regelalogrithmus der Blutvolumenregelung für den Patienten keine Risiken bestehen, die größer als die bei den Standardtherapien vorhandenen Risiken sind, bei denen der Arzt die Verantwortung für die gewählte Ultrafiltrationsrate oder das Ultrafiltrationsprofil übernimmt. Daher ist bisher bei den kommerziell erhältlichen Geräten davon abgesehen worden, die Ultrafiltrationsrate in Abhängigkeit von dem Blutvolumen des Patienten zu regeln.

Die DE-A-40 24 434 beschreibt eine extrakorporale Blutbehandlungsvorrichtung mit einer Ultrafiltrationseinrichtung. Die bekannte Blutbehandlungsvorrichtung verfügt über eine Meßeinheit zum Bestimmen des Blutvolumens, wobei die Blutvolumenbestimmung auf der Grundlage der Messung des Drucks im extrakorporalen Blutkreislauf erfolgt. Die Blutbehandlungsvorrichtung erlaubt die Vorgabe einer bestimmten Blutvolumenabnahme während der Behandlung.

Die Ultrafiltration wird so gesteuert, daß die Differenz zwischen der vorgegebenen und der gemessenen Blutvolumenabnahme minimiert wird. Dabei soll ein konventionelles UF-Kontrollsystem die Aufgabe eines Schutzsystems übernehmen und die Überschreitung einer bestimmten UF-Rate verhindern können.

Der Erfindung liegt die Aufgabe zugrunde, ein Verfahren zur Erhöhung der Sicherheit für eine extrakorporale Blutbehandlungsvorrichtung zu schaffen. Die Lösung dieser Aufgabe erfolgt erfindungsgemäß mit den im Patentanspruch 1 angegebenen Merkmalen.

Die Sicherheit des Patienten wird dadurch erhöht, dass die von der Regeleinheit der extrakorporalen Blutbehandlungsvorrichtung in Abhängigkeit von dem Blutvolumen vorgegebene Ultrafiltrationsrate auf einen oberen Grenzwert $UFR_{lim}$ limitiert wird, der aus dem vorgegebenen Ultrafiltratgesamtvolumen $UFV_{ges}$ und der vorgegebenen Behandlungsdauer UFT bestimmt wird. Die Orientierung des oberen Grenzwertes an der Ultrafiltrationsrate und der Behandlungsdauer schafft eine hohe Sicherheit für den Patienten, ohne dass der Bereich, in dem die Ultrafiltrationsrate verändert werden kann, zu stark beschränkt wird.

Bei einer bevorzugten Ausführungsform wird durch Bilden des Quotienten aus dem vorgegebenen Ultrafiltratgesamtvolumen $UFV_{ges}$ und der vorgegebenen Behandlungsdauer UFT die mittlere Ultrafiltrationsrate UFRM bestimmt, die zum Entziehen des vorgegebenen Ultrafiltratgesamtvolumens $UFV_{ges}$ innerhalb der Behandlungsdauer UFT erforderlich ist. Der obere Grenzwert $UFR_{lim}$ für die Ultrafiltrationsrate UFR wird dann durch Multiplikation der mittleren Ultrafiltrationsrate UFRM mit einem Faktor $\alpha$ bestimmt. Der Faktor $\alpha$ ist vorzugsweise größer als 1,5 und kleiner als 2,3.

Eine weitere bevorzugte Ausführungsform sieht vor, den Bereich zulässiger Ultrafiltrationsraten zum Ende der Behandlung hin zunehmend zu verkleinern. Eine Verringerung des oberen Grenzwertes für die Ultrafiltrationsrate mit zunehmender Behandlungsdauer ist insbesondere dann vorteilhaft, wenn Regelalgorithmen Verwendung finden, die in Abhängigkeit von dem Blutvolumen am Anfang der Behandlung tendenziell größere Ultrafiltrationsraten als am Behandlungsende vorgeben, so dass die Ultrafiltrationstoleranz erfahrungsgemäß zu Beginn der Behandlung größer als am Behandlungsende ist. Die Verringerung des oberen Grenzwertes für die Ultrafiltrationsrate kann eine explizite Funktion der vergangenen Behandlungszeit oder der bereits ultrafiltrierten Flüssigkeitsmenge sein.

In bevorzugter Ausgestaltung fällt der obere Grenzwert für die Ultrafiltrationsrate während der Behandlungsdauer von einem Wert, der durch Multiplikation der mittleren Ultrafiltrationsrate mit einem Faktor $\alpha$ bestimmt wird, auf die mittlere Ultrafiltrationsrate ab, wobei der Faktor $\alpha$ vorteilhafterweise zwischen 1,5 und 2,3 liegt.

In einer weiteren bevorzugten Ausgestaltung orientiert sich der obere Grenzwert für die Ultrafiltrationsrate $UFR_{lim}$ daran, dass größere Ultrafiltrationsraten dann nicht mehr zugelassen werden sollen, wenn nur noch ein geringes Restvolumen zu entziehen ist. Hierzu wird die Restultrafiltrationsrate $UFRR (t)$ bestimmt, mit der das Ultrafiltratvolumen zu einem bestimmten Zeitpunkt in dem noch zur Verfügung stehenden Zeitraum der Behandlungsdauer UFT entzogen werden kann. Durch Multiplikation der Restultrafiltrationsrate mit einem Faktor $\alpha$, der vorteilhafterweise zwischen 1,5 und 2,3 liegt, wird dann der obere Grenzwert $UFR_{lim} (t)$ zu diesem Zeitpunkt bestimmt. Die Orientierung des oberen Grenzwertes für die Ultrafiltrationsrate an der Restultrafiltrationsrate führt dazu, dass nur dann größere Raten zugelassen werden, wenn diese auch zum Entzug des Restvolumens nötig sind. Die Limitierung wirkt sich im Vergleich zu einem linearen Abfall des oberen Grenzwertes für die Ultrafiltrationsrate insbesondere dann aus, wenn schon ein Großteil des Volumens entzogen ist, also in der kritischen Phase der Annäherung an das Trockengewicht. Es wird hier eine zusätzliche Reduktion der maximal erlaubten Rate bewirkt.

Die Überwachung der von der Regeleinheit der Blutbehandlungsvorrichtung vorgegebenen Ultrafiltrationsrate hat nicht nur zum Ziel, dem Patienten vor unphysiologisch hohen Ultrafiltrationsraten zu schützen, sondern auch dafür Sorge zu tragen, dass der Patient das angestrebte Trockengewicht erreicht. Wenn die Ultrafiltrationsrate auf einen oberen Grenzwert limitiert wird, besteht die Möglichkeit, dass innerhalb der vorgegebenen Behandlungsdauer das zu filtrierende Volumen nicht entzogen werden kann.

Bei einer weiteren bevorzugten Ausführungsform wird die Restultrafiltrationsrate $UFRR(t)$ mit dem oberen Grenzwert $UFR_{lim}$ und/oder mit der mittleren Ultrafiltrationsrate UFRM verglichen. Nach dem Überschreiten der mittleren Ultrafiltrationsrate UFRM und/oder des oberen Grenzwertes $UFR_{lim}$ wird ein Warnsignal abgegeben, mit dem das Pflegepersonal darauf hingewiesen wird, dass Schwierigkeiten im Erreichen des angestrebten Trockengewichts des Patienten bestehen. Der Anwender hat dann die Möglichkeit, die Behandlungsdauer zu verlängern und/oder das Zielvolumen zu senken oder kleinere als ursprünglich geplante Blutvolumina zu tolerieren oder die Behandlung ohne Blutvolumenregelung mit der verbleibenden mittleren Ultrafiltrationsrate zu Ende zu führen.

Da die Limitierung für die Ultrafiltrationsrate nur beim Überschreiten eines unteren Grenzwertes vorgenommen werden sollte, wird vorzugsweise die von der Regeleinheit vorgegebene Ultrafiltrationsrate mit dem unteren Grenzwert verglichen. Es wird nur dann eine Beschränkung der Ultrafiltrationsrate auf den oberen Grenzwert $UFR_{lim}$ vorgenommen, wenn die Ultrafiltrationsrate größer als der untere Grenzwert ist.

Im folgenden werden mehrere Ausführungsbeispiele der Erfindung unter Bezugnahme auf die Zeichnungen näher erläutert.

Es zeigen:

Figur 1      eine Hämodiafiltrationsvorrichtung zusammen mit der Sicherheitsvorrichtung in schematischer Darstellung, die nach dem erfindungsgemäßen Verfahren arbeitet.

Figur 2      den Programmablaufplan, nach dem die Überwachungseinheit der Sicherheitsvorrichtung arbeitet,

Figur 3      den oberen Grenzwert für die Ultrafiltrationsrate $UFR_{lim}$ als Funktion der Behandlungszeit t einer Ausführungsform der Sicherheitsvorrichtung, bei der der obere Grenzwert zum Behandlungsende hin abnimmt,

Figur 4      das Ultrafiltratvolumen UFV als Funktion der Behandlungszeit t im ungünstigsten Fehlerfall unter Ultrafiltrationsratenbegrenzung gemäß der Ausführungsform von Figur 3,

Figur 5      das Ultrafiltratvolumen UFV als Funktion der Behandlungszeit t im ungünstigsten Fehlerfall bei einer weiteren Ausführungsform und

Figur 6      die Ultrafiltrationsrate UFR, die mittlere Ultrafiltrationsrate UFRM und die Restultrafiltrationsrate UFRR als Funktion der Behandlungszeit t.

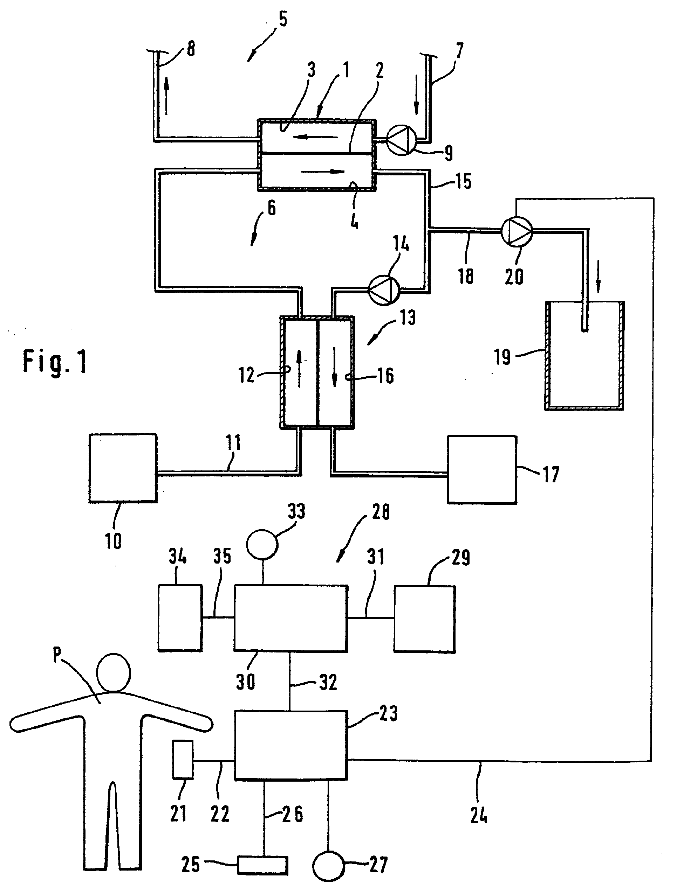

Figur 1 zeigt die wesentlichen Komponenten einer Hämodiafiltrationsvorrichtung zusammen mit einer Sicherheitsvorrichtung in schematischer Darstellung, die nach dem erfindungsgemäßen Verfahren arbeitet. Die Sicherheitsvorrichtung kann Bestandteil der Hämodiafiltrationsvorrichtung oder eine separate Einheit sein, die an eine bestehende Hämodiafiltrationsvorrichtung angeschlossen wird.

Die Hämodiafiltrationsvorrichtung umfaßt einen Dialysator 1, der durch eine semipermeable Membran 2, in eine Blutkammer 3 und eine Dialysierflüssigkeitskammer 4 geteilt ist. Die Blutkammer 3 ist in einen extrakorporalen Blutkreislauf 5 und die Dialysierflüssigkeitskammer 4 in einen Dialysierflüssigkeitskreislauf 6 geschaltet. Von dem Patienten führt eine Blutzuführleitung 7 zu dem Einlaß der Blutkammer 3 des Dialysators 1 und von dem Auslaß der Blutkammer führt eine Blutabführleitung 8 zurück zum Patienten. In die Blutzuführleitung 7 ist eine Blutpumpe 9 geschaltet.

Von einer Dialysierflüssigkeitsquelle 10 führt eine Dialysierflüssigkeitszuführleitung 11 zu dem Einlaß der ersten Bilanzkammer 12 einer Bilanziereinheit 13 und von deren Auslaß zu dem Einlaß der Dialysierflüssigkeitskammer 4 des Dialysators 1.

Eine Dialysierflüssigkeitsabführleitung 15 führt von dem Auslaß der Dialysierflüssigkeitskammer zu der zweiten Bilanzkammer 16 der Bilanziereinheit 13 und von deren Auslaß zu einem Abfluß 17. In die Dialysierflüssigkeitsabführleitung 15 ist stromauf der Bilanziereinheit 13 eine Dialysierflüssigkeitspumpe 14 geschaltet.

Stromauf der zweiten Bilanzkammer 16 der Bilanziereinheit 13 zweigt von der Dialysierflüssigkeitsabführleitung 15 eine Ultrafiltrationsleitung 18 ab, die zu einem Ultrafiltratbehälter 19 führt. Zum Abziehen des Ultrafiltrats ist in die Ultrafiltratleitung 18 eine Ultrafiltratpumpe 20 geschaltet.

Solange die Ultrafiltratpumpe 20 still steht, verhindert die Bilanziereinheit 13 einen Netto-Austausch von Flüssigkeit zwischen Primär- und Sekundärkreislauf 5, 6. Eine Ultrafiltration findet unter diesen Umständen nicht statt. Die Ultrafiltration wird erst durch das Einschalten der Ultrafiltratpumpe in Gang gesetzt, die aus der Dialysierflüssigkeitskammer 4 des Dialysators 1 gezielt Flüssigkeit entzieht (Ultrafiltration).

Die Blutbehandlungsvorrichtung verfügt über eine Meßeinheit 21 zum Bestimmen des Blutvolumens BV des Patienten P während der Hämodiafiltration. Die Meßeinheit 21 ist über eine Datenleitung 22 mit einer Steuereinheit 23 verbunden, die über eine Datenleitung 24 mit der Ultrafiltratpumpe 20 verbunden ist. Im allgemeinen ist es für die erfindungsgemäße Sicherheitsvorrichtung bzw. zur Durchführung des erfindungsgemäßen Verfahrens ausreichend, wenn die Meßeinheit 21 relative Angaben über das Blutvolumen des Patienten P zur Verfügung stellt, wie z.B. Angaben über den Wasseranteil im extrakorporalen Blut des Patienten in der Leitung 7. Es können auch andere mit dem relativen Blutvolumen korrelierende Meßgrößen gemessen werden, wie z.B. die Hämoglobin- oder Proteinkonzentration. Hierzu kann ein Ultraschallsensor Anwendung finden.

Zur Eingabe des Ultrafiltratgesamtvolumens $UFV_{ges}$, das während der gesamten Behandlungsdauer entzogen werden soll, und zur Eingabe der Behandlungsdauer UFT sowie ggf. weiterer patientenspezifischer Parameter ist eine Eingabeeinheit 25 vorgesehen, die über eine Datenleitung 26 mit der Regeleinheit 23 verbunden ist. Die Regeleinheit 23 stellt in Abhängigkeit von dem mit der Meßeinheit 21 gemessenen Blutvolumen BV des Patienten P die Ultrafiltrationsrate UFR(t) der Ultrafiltratpumpe 20 so ein, dass während der vorgegebenen Behandlungsdauer UFT das vorgegebene Ultrafiltratgesamtvolumen $UFV_{ges}$ entzogen wird (Blutvolumenregelung). Hierzu verfügt die Regeleinheit über einen entsprechenden Regelalgorithmus.

Durch Betätigung eines Schalters 27 kann die Regeleinheit 23 deaktiviert und zu einer manuellen Einstellung der Ultrafiltrationsrate übergegangen werden.

Die Sicherheitsvorrichtung 28 verhindert eine Gefährdung des Patienten bei einer fehlerhaften Blutvolumenregelung. Die Sicherheitsvorrichtung umfaßt eine Recheneinheit 29 und eine Überwachungseinheit 30, die über eine Datenleitung 31 miteinander kommunizieren. Die Überwachungseinheit ist über eine Datenleitung 32 mit der Regeleinheit 23 verbunden.

Die Recheneinheit 29 der Sicherheitsvorrichtung 28 bestimmt aus dem vorgegebenen Ultrafiltratgesamtvolumen $UFV_{ges}$ und der vorgegebenen Behandlungsdauer UFT einen oberen Grenzwert für die Ultrafiltrationsrate $UFR_{lim}$.

Durch Betätigung eines Schalters 33 kann die Sicherheitsvorrichtung 28 durch den Anwender deaktiviert werden. Dies ist insbesondere dann sinnvoll, wenn beispielsweise zum Messen von physiologisch relevanten Parametern eine kurzzeitige Erhöhung der Ultrafiltrationsrate erforderlich ist (Ultrafiltrations-Bolus). Dieses Bolusvolumen kann wiederum auf einen oberen Grenzwert beschränkt werden, der von der Überwachungseinheit vorgegeben wird, um die Sicherheit weiter zu erhöhen.

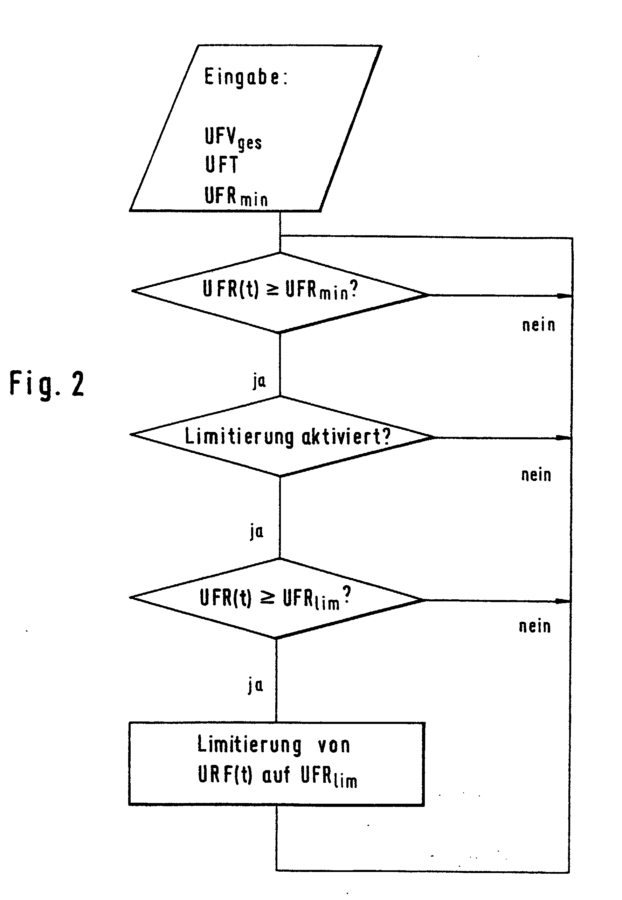

Figur 2 zeigt den Programmablaufplan, nach dem die Überwachungseinheit 29 der Sicherheitsvorrichtung 28 arbeitet.

Zu Beginn der Behandlung werden über die Eingabeeinheit 25 der Hämodiafiltrationsvorrichtung das Ultrafiltratgesamtvolumen $UFV_{ges}$, die Behandlungsdauer UFT und eine minimale Ultrafiltrationsrate $UFR_{min}$ eingegeben. Hierzu kann die Überwachungseinheit auch über eine eigene Eingabeeinheit verfügen. Die patientenspezifischen Parameter $UFV_{ges}$, UFT und $UFR_{min}$ sowie die von der Regeleinheit 23 vorgegebene Ultrafiltrationsrate UFR(t) werden der Überwachungseinheit 29 übermittelt.

Die Überwachungseinheit 29 weist eine Vergleichseinrichtung auf, mit der die von der Regeleinheit 23 vorgegebene Ultrafiltrationsrate UFR(t) mit dem unteren Grenzwert für die Ultrafiltrationsrate $UFR_{min}$ verglichen wird. Ist

UFR(t) kleiner als UFR$_{min}$ findet eine Limitierung der Ultrafiltrationsrate nicht statt. Ist UFR(t) größer als UFR$_{min}$, prüft die Überwachungseinheit, ob die Limitierung der Ultrafiltrationsrate durch den Anwender deaktiviert wurde. Ist die Limitierung aktiviert, bestimmt die Recheneinheit 29 einen oberen Grenzwert für die Ultrafiltrationsrate UFR$_{lim}$, der in der Überwachungseinheit mit der von der Regeleinheit vorgegebenen Ultrafiltrationsrate UFR(t) verglichen wird. Ist UFR(t) größer als UFR$_{lim}$, gibt die Überwachungseinheit als Ultrafiltationsrate UFR(t) den oberen Grenzwert UFR$_{lim}$ vor. Erst wenn die von der Regeleinheit vorgegebene Ultrafiltrationsrate unter den oberen Grenzwert abfällt, beendet die Überwachungseinheit 29 ihren Eingriff in die Regeleinheit 23.

Nachfolgend wird die Bestimmung des oberen Grenzwertes für die Ultrafiltrationsrate UFR$_{lim}$ im einzelnen beschrieben.

Die Recheneinheit 29 der Sicherheitsvorrichtung 28 berechnet aus dem vorgegebenen Ultrafiltratgesamtvolumen UFV$_{ges}$ und der vorgegebenen Behandlungsdauer UFT die mittlere Ultrafiltrationsrate UFRM nach der folgenden Gleichung

$$UFRM = UFV_{ges}/UFT.$$

Bei einer bevorzugten Ausführungsform berechnet die Recheneinheit aus der mittleren Ultrafiltrationsrate UFRM den oberen Grenzwert UFR$_{lim}$ wie folgt:

$$UFR_{lim} = \alpha\_UFRM,$$

wobei $1{,}5<\alpha<2{,}3$ ist.

Dieser Grenzwert ist während der Behandlungsdauer konstant. Während der Behandlungsdauer kann der obere Grenzwert UFR$_{lim}$ aber auch auf die mittlere Ultrafiltrationsrate UFRM abfallen.

Bei einer weiteren bevorzugten Ausführungsform berechnet die Recheneinheit den oberen Grenzwert UFR$_{lim}$ nach der folgenden Gleichung:

$$UFR_{lim}(t,\alpha) = [\alpha \cdot UFRM-(\alpha-1)\cdot UFRM \cdot \frac{t}{UFT}]\cdot \Phi(1-\frac{t}{UFT})+UFRM\cdot \Phi(\frac{t}{UFT}-1),$$

wobei $\Phi(t) = 0$ für $t<0$ und $\Phi(t) = 1$ für $t\geq0$ ist.

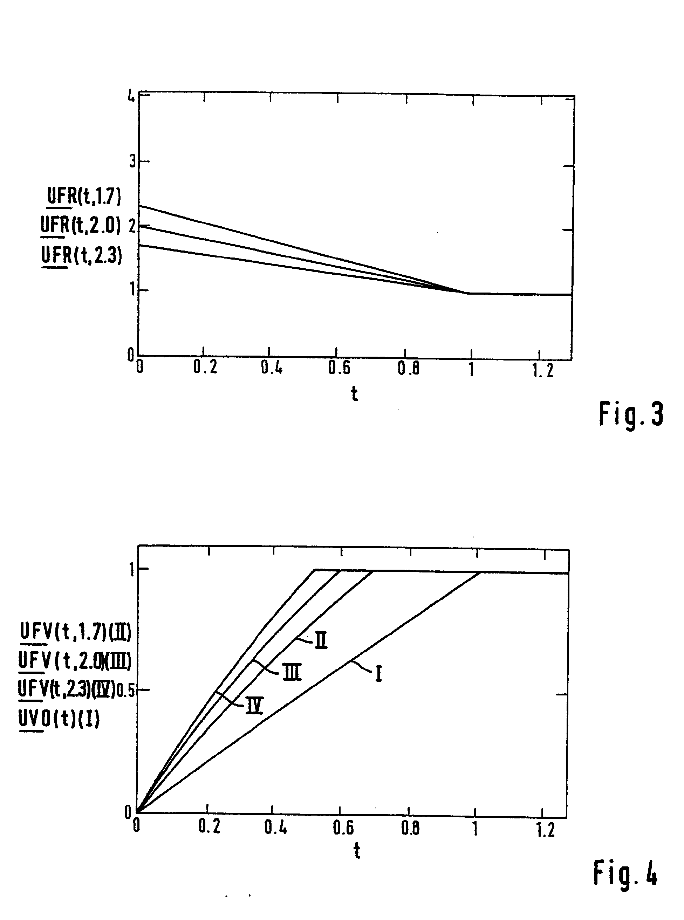

Figur 3 zeigt den oberen Grenzwert der Ultrafiltrationsrate UFR$_{lim}$(t, $\alpha$) als Funktion der Behandlungszeit t, wobei die Ordinatenwerte auf die mittlere Ultrafiltrationsrate UFRM und die Abzissenwerte auf die Behandlungsdauer UFT bezogen sind. Die drei Kurven unterscheiden sich durch den Parameter $\alpha$ = UFR$_{lim}$(0, $\alpha$)/UFRM. Zu Beginn der Behandlung werden höhere Ultrafiltrationsraten zugelassen (2 bis 2,5 UFRM), gegen Ende der Behandlung wird höchstens die mittlere Ultrafiltrationsrate UFRM zugelassen. Die Wahl eines geeigneten Parameters $\alpha$ wird durch den Variationsbereich für die Ultrafiltrationsrate bestimmt, den der Regelalgorithmus benötigt, mit dem die Regeleinheit 23 arbeitet.

Im ungünstigsten Fehlerfall gibt die Blutvolumenregelung ständig den gerade maximal zulässigen Sollwert nach Figur 3 vor, ohne dass die zugrunde liegende Fehlerursache detektiert wird. Dann stellt sich folgender Verlauf des ultrafiltrierten Volumens als Funktion der Zeit ein:

$$UFV(t,\alpha) = \alpha \cdot UFRM \cdot t-(\alpha-1)\cdot \frac{t^2}{2\cdot UFT}$$

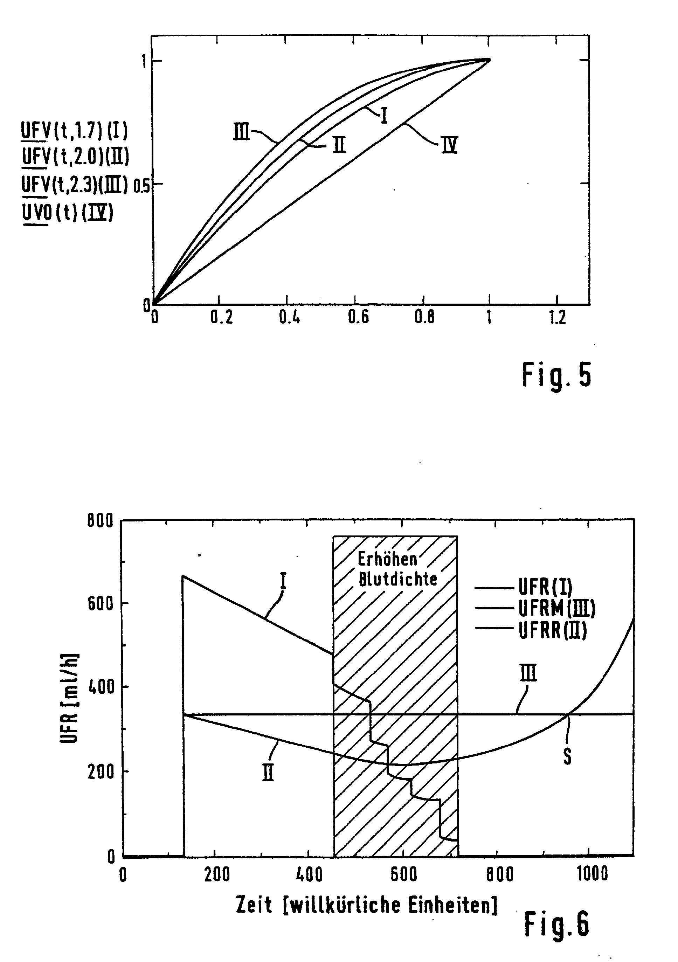

Die Kurve I in Figur 4 gibt das ultrafiltrierte Volumen bei konstanter Ultrafiltrationsrate (UFR=UFRM) an, wobei die Ordinatenwerte auf das Ultrafiltratgesamtvolumen UFV$_{ges}$ und die Abzissenwerte auf die Behandlungsdauer UFT normiert sind. Im ungünstigsten Fehlerfall steigt das Ultrafiltratvolumen UFV(t, $\alpha$) nach einer der Kurven II, III, IV (abhängig von $\alpha$) in etwa der halben Zeit auf den Endwert 1 an.

Bei einer weiteren bevorzugten Ausführungsform orientiert sich der obere Grenzwert für die Ultrafiltrationsrate UFR$_{lim}$ an der Restultrafiltrationsrate UFRR(t), die in der Recheneinheit nach der folgenden Gleichung berechnet wird:

$$UFRR(t, \alpha) = (UFV_{ges}-UFV(t)) / (UFT-t)$$

Aus der Restultrafiltrationsrate UFRR(t) berechnet die Recheneinheit den oberen Grenzwert wie folgt:

$$UFR_{lim}(t, \alpha) = \alpha\_UFRR(t),$$

wobei $1<\alpha<2,3$ ist.

Figur 5 zeigt das Ultrafiltratvolumen UFV$(t, \alpha)$ als Funktion der Behandlungszeit t, wobei die Ordinatenwerte auf das Ultrafiltratgesamtvolumen UFV$_{ges}$ und die Abzissenwerte auf die Behandlungsdauer UFT normiert sind. In Figur 5 ist der im Vergleich zu Figur 4 noch deutlich flachere Anstieg des Ultrafiltratvolumens UFV$(t, \alpha)$ zu erkennen, der sich unter den ungünstigsten Bedingungen beispielsweise bei unerkanntem Ausfall der Blutvolumenregelung zu Beginn der Behandlung ergibt, wenn eine Limitierung der Ultrafiltrationsrate durch die Überwachungseinheit erfolgt.

Neben den obigen Komponenten weist die Sicherheitsvorrichtung noch eine Warneinrichtung 34 auf, die über eine Datenleitung 35 mit der Überwachungseinheit 30 verbunden ist und über zwei akustische und/oder optische Warnsignale verfügt.

Die Warneinrichtung umfaßt eine Vergleichseinrichtung, die die Restultrafiltrationsrate UFRR(t) mit der mittleren Ultrafiltrationsrate UFRM vergleicht. Nach dem Überschreiten der mittleren Ultrafiltrationsrate UFRM gibt die Warneinrichtung ein akustisches und/oder optisches Warnsignal ab, das den Anwender darauf aufmerksam macht, dass innerhalb der Behandlungdauer UFT das vorgegebene Ultrafiltratgesamtvolumen UFV$_{ges}$ möglicherweise nicht entzogen und das Trockengewicht möglicherweise nicht erreicht werden kann.

Die Regeleinheit 23 stellt die Ultrafiltrationsrate UFR(t) so ein, dass UFR(t) größer als die mittlere Ultrafiltrationsrate UFRM ist. Unter normalen Bedingungen verläuft die Restultrafiltrationsrate UFRR(t) dann für die gesamte Behandlungsdauer unterhalb von UFRM. Das Warnsignal wird entweder unmittelbar nach dem Überschreiten der mittleren Ultrafiltrationsrate UFRM, nach Ablauf einer vorbestimmten Zeitdauer von beispielsweise 5 Minuten, nach Überschreiten einer bestimmten Toleranzgrenze UFRM$+\Delta$UFR oder nach Überschreiten einer bestimmten integrierten Ultrafiltrationsmenge abgegeben.

Die Vergleichseinrichtung der Warneinrichtung 34 vergleicht die Restultrafiltrationsrate UFRR(t) auch mit dem oberen Grenzwert der Ultrafiltrationsrate UFR$_{lim}$. Wenn die Restultrafiltrtionsrate UFRR größer als der obere Grenzwert UFR$_{lim}$ ist, gibt die Warneinrichtung ein anderes akustisches und/oder optisches Warnsignal ab, das den Anwender darauf hinweist, dass das Trockengewicht bei aktivierter Überwachungseinheit nicht mehr erreicht werden kann.

Figur 6 zeigt den Fall, dass die Regeleinheit beispielsweise aufgrund eines Fehlers während der Behandlung (schraffierter Bereich) eine sehr geringe Ultrafiltrationsrate UFR vorgibt (Kurve I). In diesem Fall steigt die Restultrafiltrationsrate UFRR an, die zum Entzug des noch verbleibenden Volumens in der restlichen Behandlungszeit notwendig ist (Kurve II). Der Schnittpunkt zwischen der Restultrafiltrationsrate UFRR und der mittleren Ultrafiltrationsrate UFRM (Kurve III) ist in Figur 6 mit S bezeichnet.

## Patentansprüche

1. Verfahren zur Erhöhung der Sicherheit für eine extrakorporale Blutbehandlungsvorrichtung, die aufweist
eine Blutzuführleitung, die von dem Patienten zu dem Einlaß einer ersten Kammer einer durch eine semipermeable Membran in die erste und eine zweite Kammer getrennte Austauscheinheit führt, und einer Blutabführleitung, die von einem Auslaß der ersten Kammer zum Patienten führt,
einer Ultrafiltrationseinrichtung zum Entziehen eines vorgegebenen Ultrafiltratgesamtvolumens UFV$_{ges}$ aus der zweiten Kammer der Austauscheinheit mit einer Ultrafiltrationsrate UFR(t) während einer vorgegebenen Behandlungsdauer UFT,
einer Meßeinheit zum Bestimmen des Blutvolumens BV oder einer mit dem Blutvolumen korrelierenden Meßgröße des Patienten und
einer Regeleinheit zur Vorgabe einer Ultrafiltrationsrate UFR(t) in Abhängigkeit von dem Blutvolumen BV, wobei
ein oberer Grenzwert für die Ultrafiltrationsrate UFR(t) vorgegeben wird,
**dadurch gekennzeichnet, dass** der obere Grenzwert für die Ultrafiltrationsrate UFR(t) aus dem vorgegebenen Ultrafiltratgesamtvolumen UFV$_{ges}$ und der vorgegebenen Behandlungsdauer UFT bestimmt wird.

2. Verfahren nach Anspruch 1, **dadurch gekennzeichnet, dass** durch Bilden des Quotienten aus dem vorgegebenen Ultrafiltratgesamtvolumen UFV$_{ges}$ und der vorgegebenen Behandlungsdauer UFT die mittlere Ultrafiltrationsrate UFRM, die zum Entziehen des vorgegebenen Ultrafiltratgesamtvolumens UFV$_{ges}$ innerhalb der Behandlungsdauer UFT erforderlich ist, und durch Multiplikation der mittleren Ultrafiltrationsrate UFRM mit einem Faktor $\alpha$ der obere Grenzwert UFR$_{lim}$ bestimmt wird.

3. Verfahren nach Anspruch 2, **dadurch gekennzeichnet, dass** der obere Grenzwert für die Ultrafiltrationsrate

UFR$_{lim}$(t) mit zunehmender Behandlungsdauer abnimmt.

4. Verfahren nach Anspruch 2, **dadurch gekennzeichnet, dass** der obere Grenzwert für die Ultrafiltrationsrate UFR$_{lim}$(t) mit zunehmenden Ultrafiltratvolumen UFV abnimmt.

5. Verfahren nach Anspruch 3 oder 4, **dadurch gekennzeichnet, dass** durch Bilden des Quotienten aus dem vorgegebenen Ultrafiltratgesamtvolumen UFV$_{ges}$ und der vorgegebenen Behandlungsdauer UFT die mittlere Ultrafiltrationsrate UFRM, die zum Entziehen des vorgegebenen Ultrafiltratgesamtvolumens UFV$_{ges}$ innerhalb der Behandlungsdauer UFT erforderlich ist und nach der folgenden Gleichung der obere Grenzwert UFR$_{lim}$(t, $\alpha$) zu einem bestimmten Zeitpunkt t bestimmt wird:

$$UFR_{lim}(t,\alpha) = [\alpha \cdot UFRM - (\alpha-1) \cdot UFRM \cdot \frac{t}{UFT}] \cdot \Phi(1-\frac{t}{UFT}) + UFRM \cdot \Phi(\frac{t}{UFT}-1)$$

wobei $\Phi$(t)=0 für t<0 und $\Phi$(t)=1 für t≥0 ist.

6. Verfahren nach Anspruch 1, **dadurch gekennzeichnet, dass** aus dem vorgegebenen Ultrafiltratgesamtvolumen UFV$_{ges}$ und der vorgegebenen Behandlungsdauer UFT nach der folgenden Gleichung

$$UFRR(t) = (UFV_{ges}-UFV(t)) / (UFV-t)$$

die Restultrafiltrationsrate UFRR(t) bestimmt wird, mit der das Ultrafiltratvolumen UFV zu einem bestimmten Zeitpunkt in dem noch zur Verfügung stehenden Zeitraum der Behandlungsdauer UFT entzogen werden kann, und dass durch Multiplikation der Restultrafiltrationsrate UFRR(t) mit einem Faktor $\alpha$ der obere Grenzwert UFR$_{lim}$(t, $\alpha$) zu diesem Zeitpunkt t bestimmt wird.

7. Verfahren nach Anspruch 6, **dadurch gekennzeichnet, dass** die Restultrafiltrationsrate UFRR(t) mit der mittleren Ultrafiltrationsrate UFRM verglichen wird, wobei nach Überschreiten der mittleren Ultrafiltrationsrate UFRM ein Warnsignal abgegeben wird.

8. Verfahren nach Anspruch 6, **dadurch gekennzeichnet, dass** die Restultrafiltrationsrate UFRR(t) mit dem oberen Grenzwert UFR$_{lim}$ für die Ultrafiltrationsrate verglichen wird, wobei nach Überschreiten des oberen Grenzwertes UFR$_{lim}$ ein Warnsignal abgegeben wird.

9. Verfahren nach einem der Ansprüche 1 bis 8, **dadurch gekennzeichnet, dass** die vorgegebene Ultrafiltrationsrate UFR(t) mit einem unteren Grenzwert für die Ultrafiltrationsrate verglichen wird, wobei die Ultrafiltrationsrate UFR(t) nur dann auf den oberen Grenzwert UFR$_{lim}$ begrenzt wird, wenn die Ultrafiltrationsrate UFR größer als der untere Grenzwert ist.

Fig.1

Eingabe:

$UFV_{ges}$
$UFT$
$UFR_{min}$

$UFR(t) \geq UFR_{min}$? — nein

ja

Limitierung aktiviert? — nein

ja

$UFR(t) \geq UFR_{lim}$? — nein

ja

Limitierung von $URF(t)$ auf $UFR_{lim}$

Fig. 2

Fig. 3

Fig. 4

UFV(t,1.7) (I)
UFV(t,2.0)(II)
UFV(t,2.3)(III)
UV0(t)(IV)

Fig. 5

Fig.6